# FASCICULE DE BREVET EUROPEEN

(11) **EP 0 637 440 B1**
(45) Date de publication et mention de la délivrance du brevet: **15.10.1997**
(21) Numéro de dépôt: 94420224.1
(22) Date de dépôt: 01.08.1994
(51) Int. Cl.: A61F 2/44

(54) **Implant intersomatique pour colonne vertébrale**
Zwischenkörperliches Implantat für die Wirbelsäule
Interbody implant for the spine

(30) Priorité: 06.08.1993 FR 9309835
(43) Date de publication de la demande: 08.02.1995
(73) Titulaire: ADVANCED TECHNICAL FABRICATION (Société Anonyme), F-74970 Marignier (FR)
(72) Inventeur: Godefroy, Jean, F-74130 Ayze (FR); Laville, Claude, F-75016 Paris (FR); Roy-Camille, Raymond, F-75007 Paris (FR)
(74) Mandataire: Poncet, Jean-François

(56) Documents cités:
- EP-A- 0 307 241
- WO-A-87/07827
- WO-A-89/12431
- US-A- 4 349 921
- US-A- 4 501 269
- US-A- 4 936 848

## Description

La présente invention concerne les implants intersomatiques tubulaires creux pour stabilisation de la colonne vertébrale, destinés à former une cale à insérer dans des rainures préparées dans les plateaux en vis-à-vis de deux vertèbres voisines, de façon à maintenir un espace discal constant.

Un tel implant intersomatique tubulaire creux est décrit par exemple dans le document US-A-4 501 269. Il comprend une cavité interne délimitée par une paroi périphérique ouverte au moins à une première extrémité, des ouvertures supérieures et inférieures assurant une communication entre l'espace extérieur et l'espace intérieur de l'implant, et permettant le contact direct entre les plateaux vertébraux et un greffon d'os spongieux inséré dans l'implant. On réalise ainsi une greffe vertébrale intersomatique.

Une autre réalisation, décrite dans le document WO-A-89 12431, est une cage cylindrique creuse comportant des trous répartis sur sa surface externe de section circulaire et comportant également un filet hélicoïdal externe pour permettre son vissage.

Ces dispositifs s'avèrent inefficaces pour assurer une bonne soudure entre deux vertèbres successives, car la dimension des trous est nécessairement limitée, et le blocage en rotation est insuffisant.

Le document EP-A-0 307 241 décrit un autre exemple d'implant sous forme d'une cage parallélépipédique. Les arêtes longitudinales d'une telle cage risquent de léser la moelle épinière ou les racines lors de l'insertion de l'implant entre les vertèbres adjacentes.

On connaît également un implant sous forme d'une cage cylindrique délimitée par quatre montants reliant deux faces extrêmes de la cage, les montants étant orientés parallèlement aux faces opposées des vertèbres. Un tel implant s'avère inefficace pour redonner une lordose anatomique, notamment à cause de la trop faible rigidité mécanique de sa structure.

Le problème proposé par la présente invention est de définir une nouvelle structure d'implant intersomatique qui permette de réduire très sensiblement les risques de lésions nerveuses lors de la pose, qui permette d'assurer un meilleur positionnement et un meilleur blocage en rotation de l'implant entre les vertèbres, et qui assure une résistance mécanique améliorée et un bon accrochage osseux pour faciliter la fusion entre deux vertèbres adjacentes.

Un autre problème que se propose de résoudre l'invention est la correction de l'angle intervertébral, en donnant à l'implant une forme particulière permettant de retrouver une lordose rachidienne lombaire anatomique.

Pour atteindre ces objets ainsi que d'autres, l'implant intersomatique selon l'invention est tel que défini dans la revendication 1.

Selon une réalisation préférée, la paroi périphérique comprend en outre au moins un relief externe anti-rotation destiné à s'incruster dans l'os d'une vertèbre pour s'opposer à la rotation de l'implant autour de son axe longitudinal.

De préférence, la paroi périphérique présente une forme générale en tronc de cône ou en ovoïde à extrémités longitudinales tronquées, dont la grande base est constituée par la face de première extrémité et dont la petite base est constituée par la face de seconde extrémité.

Selon un mode de réalisation avantageux, les surfaces externes des faces latérales comportent un méplat, et lesdites faces latérales sont dépourvues de nervures externes annulaires.

D'autres objets, caractéristiques et avantages de la présente invention ressortiront de la description suivante de modes de réalisation particuliers, faite en relation avec les figures jointes, parmi lesquelles:
- la figure 1 est une vue en perspective de trois quart et de dessus d'un implant intersomatique selon un mode de réalisation de la présente invention ;
- la figure 2 est une vue de dessus de l'implant de la figure 1 ;
- la figure 3 est une vue de côté en coupe longitudinale montrant un implant de la figure 1 en position entre deux vertèbres ;
- la figure 4 est une coupe transversale selon le plan A-A de la figure 3 ;
- la figure 5 est une vue de côté en coupe longitudinale montrant un implant à forme générale en ovoïde à extrémités longitudinales tronquées ;
- la figure 6 est une vue en perspective de trois quart et de dessus d'un implant selon un second mode de réalisation de l'invention
- la figure 7 est une vue de dessus de l'implant de la figure 6 ;
- la figure 8 est une coupe longitudinale de l'implant de la figure 6 ; et
- la figure 9 est une coupe transversale de l'implant de la figure 6.

Comme le représentent les figures, l'implant intersomatique selon l'invention est un élément tubulaire creux présentant un axe longitudinal I-I, réalisé par exemple en métal ou en matériau synthétique rigide, comprenant une cavité interne 1 délimitée par une paroi périphérique 2 ouverte au moins à une première extrémité 3.

La paroi périphérique 2 comprend une surface externe 4 à section généralement circulaire. Cette surface externe 4 se compose de deux faces latérales 5 et 6 pleines et de deux faces respectives supérieure 7 et inférieure 8 munies de lumières de communication. Par exemple, la face supérieure 7 est munie de deux lumières de communication 9 et 10, la face inférieure 8 étant munie de deux lumières de communication 11 et 12.

La paroi périphérique 2 comprend en outre au moins une nervure externe annulaire anti recul telle que la nervure 13 voisine de la première extrémité 3.

Dans les modes de réalisation représentés, l'implant comprend une première nervure externe annulaire 13 au voisinage de sa première extrémité 3, une seconde nervure externe annulaire 14 au voisinage de sa seconde extrémité 15, et une nervure externe annulaire intermédiaire 16 ménagée sur une zone intermédiaire 17 de paroi périphérique 2 dépourvue de lumière de communication.

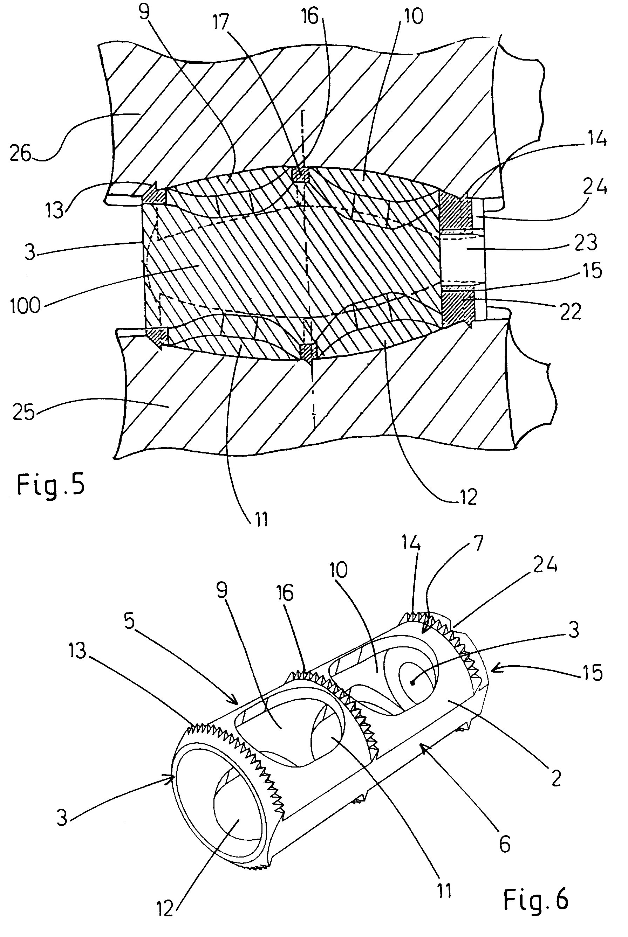

De préférence, comme illustré dans le mode de réalisation représenté sur les figures 6 à 9, les nervures externes annulaires 13, 14 et 16 sont dentées, pour s'opposer à la fois à la translation axiale et à la rotation de l'implant, les dents venant s'incruster dans les plateaux vertébraux.

En alternative, on peut prévoir une ou plusieurs nervures longitudinales faisant saillie sur la surface externe de la paroi périphérique 2. Ces moyens anti-rotation sont toutefois plus difficiles à réaliser que les dents de nervures externes annulaires.

Dans tous les modes de réalisation, les lumières de communication 9, 10, 11 et 12 ont avantageusement une forme oblongue, de longueur seulement peu inférieure à la demi-longueur de l'implant, de largeur sensiblement égale au diamètre de l'implant.

On comprendra qu'un implant selon l'invention peut comprendre une, deux ou trois nervures, ou un nombre plus important de nervures externes annulaires.

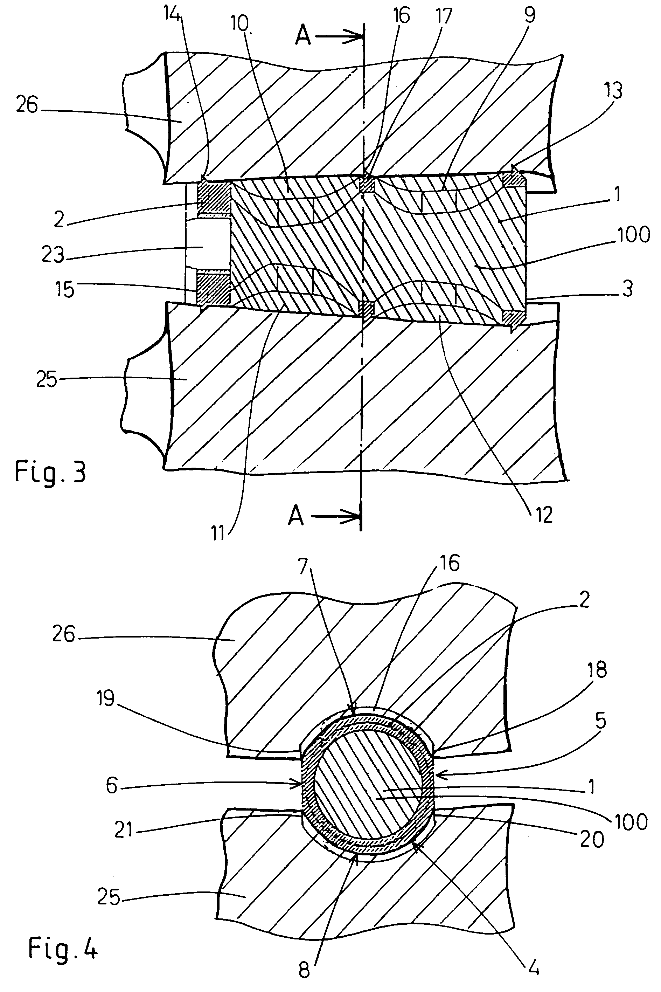

De préférence, comme le représentent notamment les figures 3, 5 et 8, chaque nervure externe annulaire 13, 14 ou 16 présente une section transversale en dent de scie asymétrique s'opposant au déplacement de l'implant en direction de la seconde extrémité 15.

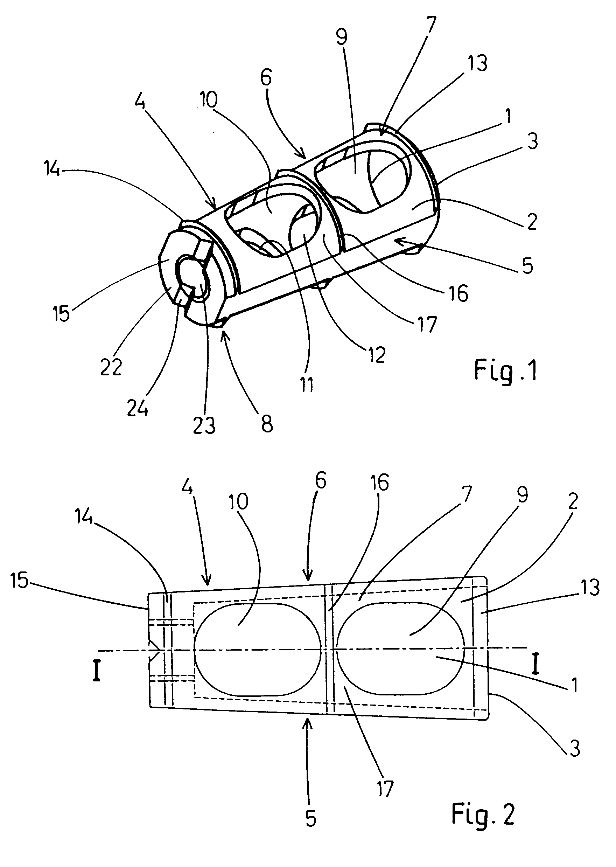

Dans les modes de réalisation des figures 1 à 3 et 6 à 8, la paroi périphérique 2 présente une forme générale externe en tronc de cône dont la grande base est constituée par la face de première extrémité 3 et dont la petite base est constituée par la face de seconde extrémité 15. Une telle forme permet de redonner et de maintenir l'angle physiologique approprié entre deux vertèbres adjacentes.

Dans le mode de réalisation de la figure 5, la paroi périphérique 2 présente une forme générale externe en ovoïde à extrémités longitudinales tronquées formant une grande base à la première extrémité 3 et une petite base à la seconde extrémité 15.

La forme ovoïde est choisie de façon à épouser plus étroitement la forme anatomique des plateaux vertébraux entre lesquels l'implant vient s'insérer. Avant insertion de l'implant, on donne alors aux rainures un profil longitudinal adapté.

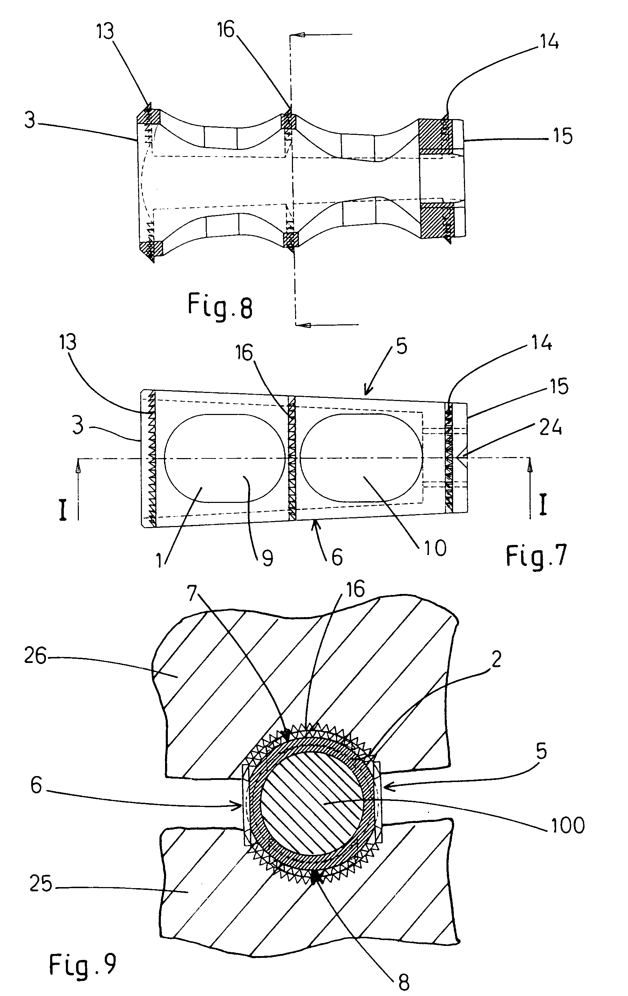

Dans les modes de réalisation représentés, les faces latérales 5 et 6 sont dépourvues de nervures externes périphériques. En conséquence, comme on le voit mieux sur les figures 4 et 9, les nervures externes annulaires sont interrompues au droit de chaque face latérales 5 ou 6. Par exemple, la nervure périphérique 16 comporte deux interruptions supérieures respectives 18 et 19, et deux interruptions inférieures respectives 20 et 21, qui s'opposent à la rotation de l'implant lorsque celui-ci est engagé entre deux vertèbres adjacentes.

Les surfaces externes des faces latérales 5 et 6 peuvent comprendre un méplat, comme représenté sur les figures 1, 4, 6 et 9, ce qui permet de réduire quelque peu la quantité de matière constituant l'implant. Le méplat occupe toute la longueur de la face latérale correspondante.

La paroi périphérique 2 se raccorde, à sa seconde extrémité 15, à une paroi transversale d'appui 22. Cette paroi transversale d'appui 22 comprend un trou axial 23 taraudé, permettant l'adaptation d'un outil de pose en prolongement de l'implant.

La paroi transversale d'appui 22 comprend en outre des reliefs externes, par exemple une rainure ou fente diamétrale 24, permettant de solidariser en rotation un instrument de pose sur l'implant.

De préférence, les reliefs externes sont conformés de façon à constituer en eux-mêmes un moyen de repérage visuel de position angulaire de l'implant autour de son axe longitudinal I-I. Une structure de relief externe en forme de rainure ou fente diamétrale convient pour constituer un tel moyen de repérage visuel de position angulaire. Cela permet en particulier de positionner l'implant entre deux vertèbres de façon que les faces supérieure 7 et inférieure 8 soit en regard des faces correspondantes des deux vertèbres adjacentes entre lesquelles l'implant est engagé.

Les figures 3, 4, 5 et 9 représentent un implant selon l'invention en position entre deux vertèbres adjacentes 25 et 26.

Préalablement à la pose de l'insert, on introduit dans sa cavité interne 1 un greffon d'os spongieux 100 prélevé dans une autre partie du corps du patient.

Avant d'engager l'implant entre les deux vertèbres, on réalise dans les plateaux respectifs de chaque vertèbre une rainure de section appropriée orientée sagittalement par rapport à l'axe longitudinal du rachis, l'ensemble des deux rainures formant sensiblement un logement cylindrique dont la section correspond sensiblement à la dimension de la seconde extrémité 15 de l'implant. On écarte ensuite les deux vertèbres 25 et 26 par des moyens appropriés, et l'on insère axialement l'implant dans les deux rainures, puis l'on relâche les vertèbres. La forme en tronc de cône de l'implant donne aux vertèbres 25 et 26 une angulation appropriée, reproduisant l'angle morphologique normal tel que la lordose lombaire anatomique. Les nervures externes annulaires 13, 14 et 16 s'engagent dans l'os formant les vertèbres 25 et 26, et interdisent le déplacement axial de l'insert. Les extrémités 18 à 21 des nervures externes annulaires, ainsi que les dents ou nervures longitudinales, en appui sur l'os empêchent également la rotation de l'insert autour de son axe longitudinal.

Après la pose, le greffon d'os spongieux 100 est en contact avec l'os des vertèbres inférieure 25 et supérieure 26, de façon à assurer progressivement la fusion des vertèbres l'une à l'autre. La taille importante des lumières de communication 9, 10, 11 et 12 favorise cette fusion, en assurant un bon contact entre le greffon interne 100 et l'os des deux vertèbres 25 et 26.

Les faces latérales pleines 5 et 6 de l'insert selon l'invention assurent une bonne rigidité de l'insert, pour reprendre les efforts de compression entre les deux vertèbres 25 et 26.

La section généralement circulaire de l'insert évite le risque de lésion neurologique lors de l'insertion de l'insert entre les deux vertèbres.

La présente invention n'est pas limitée aux modes de réalisation qui ont été explicitement décrits, mais elle en inclut les diverses variantes et généralisations contenues dans le domaine des revendications ci-après.

## Revendications

1. Implant intersomatique tubulaire creux pour stabilisation de la colonne vertébrale, comprenant une cavité interne (1) délimitée par une paroi périphérique (2) comprenant une surface externe (4) à section généralement circulaire et munie de luminières de communication (9, 10, 11, 12), caractérisé en ce que la surface externe (4) se compose de deux faces latérales (5, 6) pleines et deux faces supérieure (7) et inférieure (8) munies desdites lumières de communication (9, 10, 11, 12), et en ce que la paroi périphérique comprend au moins une nervure externe annulaire (13) anti recul pouvant être interrompue.

2. Implant selon la revendication 1, caractérisé en ce que la paroi périphérique (2) comprend en outre au moins un relief externe anti-rotation.

3. Implant selon l'une des revendications 1 ou 2, caractérisé en ce que la paroi périphérique (2) présente une forme générale en tronc de cône ou en ovoïde à extrémités longitudinales tronquées dont la grande base est constituée par la face de première extrémité (3).

4. Implant selon l'une quelconque des revendications 1 à 3, caractérisé en ce que la paroi périphérique (2) comprend une nervure externe annulaire (13, 14) respectivement au voisinage de sa première extrémité (3) et de sa seconde extrémité (15).

5. Implant selon la revendication 4, caractérisé en ce qu'il comprend en outre une nervure externe annulaire intermédiaire (16), ménagée dans une zone intermédiaire (17) de paroi dépourvue de lumière de communication.

6. Implant selon la revendication 2, caractérisé en ce que ledit relief externe anti-rotation comprend au moins une dent faisant saillie de l'une au moins des nervures externes annulaires (13) anti-recul.

7. Implant selon l'une quelconque des revendications 1 à 6, caractérisé en ce que les faces latérales (5, 6) sont dépourvues de nervures externes annulaires.

8. Implant selon l'une quelconque des revendications 1 à 7, caractérisé en ce que les surfaces externes des faces latérales (5, 6) comportent un méplat.

9. Implant selon l'une quelconque des revendications 1 à 8, caractérisé en ce que la paroi périphérique (2) se raccorde, à sa seconde extrémité (15), à une paroi transversale d'appui (22).

10. Implant selon la revendication 9, caractérisé en ce que la paroi transversale d'appui (22) comprend un trou axial (23) taraudé pour adaptation d'un instrument de pose.

11. Implant selon la revendication 10, caractérisé en ce que la paroi transversale d'appui (22) comporte des reliefs externes (24) pour solidariser en rotation l'outil de pose sur l'implant.

12. Implant selon la revendication 11, caractérisé en ce que les reliefs externes (24) sont conformés de façon à constituer un moyen de repérage visuel de position angulaire de l'implant autour de son axe longitudinal (I-I).

## Claims

1. Hollow tubular intersomatic implant for stabilization of the vertebral column, comprising an internal cavity (1) delimited by a peripheral wall (2) comprising a generally circular cross-section external surface (4) and having communicating holes (9, 10, 11, 12), characterized in that the external surface (4) is composed of two solid side surfaces (5, 6) and top (7) and bottom surfaces (8) having said communicating holes (9, 10, 11, 12) in them, and in that the peripheral wall comprises at least one annular external rib (13) that prevent retrograde movement that can be interrupted.

2. Implant according to claim 1, characterized in that the peripheral wall (2) further comprises at least one rotation preventing external relief.

3. Implant according to claim 1 or claim 2, characterized in that the peripheral wall (2) is generally frustoconical or ovoidal with truncated longitudinal ends the larger base of which consists of the surface at its first end (3).

4. Implant according to any one of claims 1 to 3, characterized in that the peripheral wall (2) comprises a respective annular external rib (13, 14) near its first end (3) and near its second end (15).

5. Implant according to claim 4, characterized in that it further comprises an intermediate annular external rib (16) in an intermediate area (17) of the wall in which there are no communicating holes.

6. Implant according to claim 2, characterized in that said rotation preventing external relief comprises at least one tooth projecting from at least one of the annular external ribs (13) for preventing retrograde movement.

7. Implant according to any one of claims 1 to 6, characterized in that the side surfaces (5, 6) have no annular external ribs.

8. Implant according to any one of claims 1 to 7, characterized in that the side surfaces (5, 6) include a flat on their outside.

9. Implant according to any one of claims 1 to 8, characterized in that the peripheral wall (2) is joined, at its second end (15), to a transverse bearing wall (22).

10. Implant according to claim 9, characterized in that the transverse bearing wall (22) comprises a threaded axial hole (23) for fitting an inserting tool.

11. Implant according to claim 10, characterized in that the transverse bearing wall (22) includes external reliefs (24) for preventing rotation of the inserting tool relative to the implant.

12. Implant according to claim 11, characterized in that the external reliefs (24) are shaped to constitute means for visually indicating the angular position of the implant about its longitudinal axis (I-I).

## Patentansprüche

1. Hohles, röhrenförmiges zwischenkörperliches Implantat zur Stabilisierung der Wirbelsäule, welches einen inneren Hohlraum (1) aufweist, der durch eine Außenwandung (2) begrenzt ist, die eine äußere Oberfläche (4) mit generell kreisförmigem Querschnitt aufweist und mit Durchtrittsöffnungen (9, 10, 11, 12) versehen ist, dadurch gekennzeichnet, daß sich die äußere Oberfläche (4) aus zwei seitlichen massiven Flächen (5, 6) und zwei oberen (7) und unteren mit besagten Durchtrittsöffnungen (9, 10, 11, 12) versehenen Flächen (8) zusammensetzt, und daß die Außenwandung mindestens eine äußere, verschiebungshemmende, ringförmige Rippe (13) umfaßt, die unterbrochen sein kann.

2. Implantat gemäß Anspruch 1, dadurch gekennzeichnet, daß die Außenwandung (2) zusätzlich mindestens eine außenliegende verdrehungshemmende Erhebung aufweist.

3. Implantat gemäß einem der Ansprüche 1 oder 2, dadurch gekennzeichnet, daß die Außenwandung (2) eine allgemeine Form eines Kegelstumpfes oder einer Eiform mit längsseitig abgestumpften Extremitäten aufweist, deren große Grundfläche von der Fläche der ersten Extremität (3) gebildet ist.

4. Implantat gemäß einem der Ansprüche 1 bis 3, dadurch gekennzeichnet, daß die Außenwandung (2) je eine äußere ringförmige Rippe (13, 14) nahe ihrer ersten Extremität (3) und ihrer zweiten Extremität (15) aufweist.

5. Implantat gemäß Anspruch 4, dadurch gekennzeichnet, daß es eine weitere äußere, ringförmige, zwischenliegende Rippe (16) aufweist, die in einem durchtrittsöffnungsfreien, mittleren Bereich (17) der Wandung angebracht ist.

6. Implantat gemäß Anspruch 2, dadurch gekennzeichnet, daß die besagte äußere, verdrehungshemmende Erhebung mindestens einen Zahn aufweist, der von der wenigstens einen äußeren, ringförmigen, verschiebungshemmenden Rippe (13) vorspringt.

7. Implantat gemäß einem beliebigem der Ansprüche 1 bis 6, dadurch gekennzeichnet, daß die seitlichen Flächen (5, 6) ohne äußere, ringförmige Erhebungen ausgeführt sind.

8. Implantat gemäß einem beliebigen der Ansprüche 1 bis 7, dadurch gekennzeichnet, daß die äußeren Oberflächen der seitlichen Flächen (5, 6) eine ebene Fläche aufweisen.

9. Implantat gemäß einem beliebigen der Ansprüche 1 bis 8, dadurch gekennzeichnet, daß die Außenwandung (2) an ihrer zweiten Extremität (15) mit einer transversalen Stützfläche (22) verbunden ist.

10. Implantat gemäß Anspruch 9, dadurch gekennzeichnet, daß die transversale Stützfläche (22) ein axiales Loch (23) mit Innengewinde zur Adaptation eines Positionierungsinstrumentes enthält.

11. Implantat gemäß Anspruch 10, dadurch gekennzeichnet, daß die transversale Stützfläche (22) äußere Profilierung (24) aufweist zum Zusammenwirken des Positionierungswerkzeuges mit dem Implantat beim Drehen.

12. Implantat gemäß Anspruch 11, dadurch gekennzeichnet, daß die äußeren Profilierungen (24) so ausgebildet sind, daß sie ein Mittel zur visuellen Kennzeichnung der Winkelposition des Implantats bezüglich seiner Längsachse (I-I) bilden.
